# FASCICULE DE BREVET EUROPEEN

(11) **EP 1 778 306 B1**
(45) Date de publication et mention de la délivrance du brevet: **29.06.2011**
(21) Numéro de dépôt: 05793512.4
(22) Date de dépôt: 25.07.2005
(51) Int. Cl.: A61L 27/10

(54) **BIOVERRE POREUX ET PROCEDE DE PREPARATION**
PORÖSES BIOGLAS UND HERSTELLUNGSVERFAHREN DAFÜR
POROUS BIOGLASS AND PREPARATION METHOD THEREOF

(30) Priorité: 27.07.2004 FR 0408288
(43) Date de publication de la demande: 02.05.2007
(73) Titulaire: Institut National Des Sciences Appliquees, F-69100 Villeurbanne (FR)
(72) Inventeur: ZENATI, Rachid, F-69250 Neuville sur Saone (FR); FANTOZZI, Gilbert, F-69330 Meyzieu (FR); CHEVALIER, Jérôme, 33 rue Louise Weiss, F-69140 Rillieux la Pape (FR); ARIOUA, Mourad, F-69100 Villeurbanne (FR)
(74) Mandataire: Brédeville, Odile Marie
(86) Numéro de dépôt international: PCT/FR2005/001921
(87) Numéro de publication internationale: WO 2006/018531

(56) Documents cités:
- EP-A- 0 401 793
- WO-A-02/096391
- US-A1- 2001 014 355
- US-A1- 2003 138 473
- LIN F-H ET AL: "FABRICATION AND BIOCOMPATIBILITY OF A POROUS BIOGLASS CERAMIC IN A SODIUM MONOXIDE CALCIUM OXIDE SILICON DIOXIDE PYROPHOSPHATE SYSTEM" JOURNAL OF BIOMEDICAL ENGINEERING, vol. 13, no. 4, 1991, pages 328-334, XP009046069 ISSN: 0141-5425

## Description

La présente invention concerne un procédé d'obtention d'une composition cristalline ou partiellement cristalline, ou bioverre, poreuse particulièrement résistante mécaniquement et utilisable comme substitut osseux.

Dans le domaine des substituts synthétiques de l'os, les implants à base d'hydroxapatite et de phosphate tricalcique sont connus et utilisés depuis de nombreuses années. L'hydroxyapatatite est une céramique de phosphate de calcium de composition proche de la partie minérale de l'os naturel. C'est elle qui permet la cicatrisation à court terme, à partir de la surface de l'implant. Toutefois, l'hydroxyapatite n'est pas résorbable et demeure à long terme dans le corps du patient. Pour limiter cet inconvénient, on lui adjoint du phosphate tricalcique qui est un phosphate de calcium résorbable permettant à l'os de prendre sa place pour une réhabilitation osseuse de l'implant à long terme.

Les implants combinant hydroxapatite et phosphate tricalcique sont ainsi considérés comme l'alternative majeure aux greffes autologues, ces dernières n'étant pas toujours possibles. Toutefois, contrairement à l'os autologue, ces implants à base d'hydroxapatite et de phosphate tricalcique ne sont pas ostéoinducteurs, c'est-à-dire qu'ils ne favorisent pas le développement des cellules osseuses au coeur de l'implant.

Un autre inconvénient des implants existants est qu'ils sont difficiles à manipuler et à mettre en oeuvre sous forme de masse. Ainsi, une masse de céramique hydroxyapatite-phosphate tricalcique est difficile à tailler pour le chirurgien à la forme voulue au moment de l'opération, car trop dure et friable.

Pour remédier à cet inconvénient, les substituts osseux à base d'hydroxyapatite sont parfois fournis sous forme de granulés. Le chirurgien remplit la cavité à combler avec ces granulés. Toutefois, cette technique est délicate et nécessite une cage de maintien pour éviter toute dissémination hors du site à traiter. Les granulés peuvent s'effriter ou causer des lésions par leurs débris.

On a donc cherché à développer des substituts osseux à base d'hydroxyapatite poreux afin de les rendre plus facilement manipulables et taillables. Toutefois, un inconvénient majeur de ces substituts osseux est leur fragilité. En effet, du fait de la présence de collagène, l'os naturel est ductile et résistant. Au contraire, les implants poreux à base d'hydroxyapatite et de phosphate tricalcique se cassent et s'effritent facilement. Ainsi, il est très difficile pour un chirurgien de découper dans une masse poreuse de substitut osseux à base d'hydroxyapatite-phosphate tricalcique une forme particulière sans que la masse ne s'effrite.

L'article « Effect of varying physical properties of porous, surface modified bioactive glass 45S5 on osteoblast proliferation and maturation », E.A.B. Effah Kaufmann, P. Ducheyne, I.M. Shapiro, Journal of biomedical materials research, 2000, vol. 52, n° 4, pp. 783-796, décrit un bioverre poreux favorisant le développement des cellules osseuses après implantation. Toutefois, ce bioverre poreux est extrêmement fragile. Il ne présente pas une bonne résistance mécanique et s'effrite lorsqu'on cherche à le tailler pour réaliser une forme particulière d'implant. L'article «Fabrication and biocompatibility of a porous bioglass ceramic in a Na₂O-CaO-SiO₂-P₂O₅ system», Feng-Huei Lin et al, décrit un procédé d'obtention d'une composition cristalline ou partiellement cristalline comprenant une étape de compactage d'un mélange verre et agent porogène.

De même, l'article « Porous bioactive glass and glass-ceramic made by reaction sintering under pressure », Weiliang Gong, Abdesselam Abdelouas, Werner Lutze, Journal of biomédical materials research, 2001, vol. 54, n° 3, pp. 320-327, décrit des verres et des vitrocéramiques préparés par frittage sous pression isostatique. Toutefois, ces compositions présentent un faible taux de porosité qui les rend difficiles à manipuler et à tailler. Par ailleurs, le développement des cellules osseuses n'est pas favorisé.

Ainsi, il existe le besoin d'un bioverre massif poreux, apte à favoriser le développement des cellules osseuses in vivo après implantation dans le corps humain en substitution d'os naturel, et présentant des propriétés mécaniques permettant de tailler ce bioverre à la forme souhaitée sans qu'il ne perde son intégrité.

La présente invention vise à remédier à ce problème en proposant une composition cristalline ou semi-cristalline poreuse à base du système SiO₂, CaO, Na₂O, et P₂O₅ comprenant des macropores et des micropores de tailles respectives particulières et un taux de porosité spécifique, et présentant une résistance en compression allant de 7 MPa à 70 MPa.

La présente invention décrit une composition cristalline ou partiellement cristalline poreuse massive comprenant au moins SiO₂, CaO, Na₂O, et P₂O₅, comprenant des micropores et des macropores, caractérisée en ce que :
- le taux de porosité va de 50% à 80% mesuré par la méthode géométrique,
- le diamètre moyen des macropores va de 100 à 1250 microns, de préférence de 150 microns à 300 microns,
- le diamètre moyen des micropores est inférieur ou égal à 5 microns,
- la résistance en compression va de 7 MPa à 70 MPa.

Un autre objet de l'invention est un implant caractérisé en ce qu'il est constitué d'une composition telle que ci-dessus.

L'objet de la présente invention est un procédé de préparation d'une composition cristalline ou partiellement cristalline poreuse massive comprenant au moins SiO₂, CaO, Na₂O, et P₂O₅, comprenant des micropores et des macropores, dont le taux de porosité va de 50% à 80% mesuré par la méthode géométrique, le diamètre moyen des macropores allant de 100 à 1250 microns, de préférence de 150 microns à 300 microns, le diamètre moyen des micropores étant inférieur ou égal à 5 microns, caractérisé en ce qu'il comprend les étapes suivantes :
- a°) on dispose d'un verre à base de SiO₂, CaO, Na₂O, et P₂O₅,
- b°) le verre de l'étape a°) est broyé et tamisé pour obtenir une poudre,
- c°) la poudre obtenue en b°) est mise en suspension dans un solvant et dispersée à l'aide d'un dispersant pour obtenir une barbotine,
- d°) un agent épaississant est rajouté à la barbotine obtenue en c°)
- e°) le mélange obtenu en d°) est coulé dans un moule puis séché pour donner un « gâteau »,
- f°) le « gâteau » obtenu en e°) est chauffé à une température d'environ 600°C pour obtenir un « cru »,
- g°) le cru obtenu en f°) est traité à une température allant de 600°C à 1100°C, de préférence de 900°C à 1100°C, afin d'obtenir une cristallisation partielle ou totale de la composition.

Un autre objet de l'invention est une composition susceptible d'être obtenue selon le procédé ci-dessus.

La composition selon l'invention présente une résistance mécanique permettant de tailler des formes particulières dans sa masse sans que cette dernière ne s'effrite ou perde son intégrité, et ce malgré son taux de porosité élevé. Ainsi, la composition selon l'invention est facilement manipulable. Le chirurgien peut simplement et facilement la tailler, éventuellement la retailler à la forme voulue avant de l'implanter.

Il est possible de tailler dans la composition selon l'invention des implants de toute forme souhaitée, par exemple pour combler un défaut osseux.

L'implant réalisé étant alors une pièce unique, il est particulièrement résistant et stable. Il ne génère pas de débris indésirables.

La composition selon l'invention présente une porosité homogène. Ceci ressort en particulier de la faible dispersion des mesures de résistance en compression de ces compositions. La présence de micropores favorise l'adhésion des cellules. La présence des macropores favorise la pénétration des cellules, en particulier des cellules de taille importante.

De plus, les implants réalisés à partir de la composition selon l'invention sont résorbables. Ainsi, ils sont tout à fait compatibles avec une utilisation in vivo en tant qu'implant, en particulier comme substitut osseux. De plus, ils favorisent le développement des cellules osseuses. A long terme, l'implant est ainsi remplacé par de l'os naturel.

Enfin, du fait de leur porosité, les implants réalisés à partir de la composition de l'invention sont particulièrement légers, ce qui est un confort pour le patient et pour le chirurgien.

Par « bioverre », on entend au sens de la présente invention un verre, partiellement ou totalement recristallisé, compatible avec le corps humain, et bioactif, c'est-à-dire capable de développer une réponse biologique à l'interface entre ledit verre et les tissus humains, et donc de développer une liaison entre ledit verre et lesdits tissus humains.

Par composition « massive », on entend au sens de la présente invention, une composition tridimensionnelle solide, dans laquelle il est possible de tailler des implants, et non une composition pulvérulente.

La présente invention va maintenant être décrite plus en détails ci-après.
Les figures 1, 2 et 3 sont des photographies, prises au microscope électronique à balayage environnemental FEI XL30, respectivement aux grossissements X101, X500 et X3000, d'une composition obtenue selon le procédé de l'invention, dans lequel le traitement de l'étape g°) a été effectué à 1100°C.
Les figures 4, 5, et 6 sont des photographies prises au microscope électronique à balayage environnemental FEI XL30, respectivement aux grossissements X101, X500 et X3000, d'une composition obtenue selon le procédé de l'invention, dans lequel le traitement de l'étape g°) a été effectué à 900°C.
Les figures 7 et 8 sont des photographies prises au microscope électronique à balayage environnemental FEI XL30, respectivement aux grossissements X103 et X5054, d'une composition obtenue selon le procédé de l'invention, dans lequel le traitement de l'étape g°) a été effectué à 1100°C, puis traitée dans une solution de liquide physiologique pendant 24h.

La composition selon l'invention comprend au moins SiO₂, CaO, Na₂O, et P₂O₅. La composition peut en outre comprendre un composant choisi parmi K₂O, MgO, Al₂O₃, CaF₂, B₂O₃ et leurs mélanges.

Dans une forme préférée de réalisation de l'invention, SiO₂ est présent dans la composition à une teneur allant de 40% à 55% en poids, par rapport au poids total de la composition, CaO est présent dans la composition à une teneur allant de 15% à 25% en poids, par rapport au poids total de la composition, Na₂O est présent dans la composition à une teneur allant de 15% à 25% en poids, par rapport au poids total de la composition et P₂O₅ est présent dans la composition à une teneur allant de 1% à 9% en poids, par rapport au poids total de la composition.

K₂O peut par exemple être présent dans la composition à une teneur allant de 0,1 à 8% en poids, par rapport au poids total de la composition. MgO peut par exemple être présent dans la composition à une teneur allant de 0,1 à 5% en poids, par rapport au poids total de la composition. Al₂O₃ peut par exemple être présent dans la composition à une teneur allant de 0,1 à 1,5% en poids, par rapport au poids total de la composition. CaF₂ peut par exemple être présent dans la composition à une teneur allant de 0,1 à 12,5% en poids, par rapport au poids total de la composition. B₂O₃ peut par exemple être présent dans la composition à une teneur allant de 0,1 à 10% en poids, par rapport au poids total de la composition.

De préférence encore, la composition est constituée de 45% en poids de SiO₂, par rapport au poids total de la composition, de 24,5% en poids de CaO, par rapport au poids total de la composition, de 24,5% en poids de Na₂O, par rapport au poids total de la composition et de 6% en poids de P₂O₅, par rapport au poids total de la composition. Une telle composition favorise particulièrement le développement des cellules osseuses.

La composition selon l'invention est poreuse et présente un taux de porosité allant de 50% à 80% mesuré par la méthode géométrique, de préférence de 60 à 75%. Dans cette méthode, le taux de porosité T est donné par la relation :
T = 1 - (masse volumique apparente/masse volumique théorique)
dans laquelle la masse volumique apparente est déterminée par la méthode géométrique. Cette masse est calculée selon la formule p = m / V dans laquelle :
   - p est la masse volumique (g/cm³),
   - m est la masse de l'échantillon (g),
   - V est le volume de l'échantillon (cm³)

La masse volumique théorique du matériau est estimée à environ 2,75 g/cm³, quelle que soit la composition en oxydes.

Le diamètre moyen des macropores va de 100 à 1250 microns, de préférence de 150 microns à 300 microns. Ainsi, les cellules de taille importante, telles que les ostéocytes, peuvent pénétrer dans l'implant à travers des interconnections de tailles suffisantes. Le diamètre moyen des micropores est inférieur ou égal à 5 microns. Ainsi, l'adhésion des cellules ainsi que la circulation des fluides biologiques sont particulièrement favorisées.

La composition selon l'invention présente une résistance en compression allant de 7 MPa à 70 MPa, de préférence de 25 MPa à 70MPa.

Dans l'ensemble de la présente demande, la résistance en compression est mesurée selon la méthode suivante : des échantillons de compositions selon l'invention sont préparés sous la forme de cylindres de diamètre 9 mm et de hauteur 6 mm. Le test de résistance en compression est réalisé à l'aide d'une machine Instron® 8512 hydraulique possédant une cellule de charge de 5000 N. On applique la charge sur l'échantillon avec une vitesse de traverse de 1 mm/min et on mesure la force nécessaire pour que l'échantillon casse. Cette force correspond à la résistance en compression de la composition.

Dans une forme préférée de réalisation de l'invention, la composition selon l'invention présente une résistance en compression, mesurée selon cette méthode, d'environ 7 MPa, pour un taux de porosité, mesuré selon la méthode géométrique, d'environ 75%.

Dans une autre forme préférée de réalisation de l'invention, la composition selon l'invention présente une résistance en compression, mesurée selon cette méthode, d'environ 70 MPa, pour un taux de porosité, mesuré selon la méthode géométrique, d'environ 50%.

Les compositions selon l'invention sont de préférence préparées par le procédé qui va être décrit ci-après.

Dans une première étape, étape a°), on dispose d'un verre à base de SiO₂, CaO, Na₂O, et P₂O₅. Le verre de l'étape a°) peut en outre comprendre un composant choisi parmi K₂O, MgO, Al₂O₃, CaF₂, B₂O₃ et leurs mélanges.

Dans une forme préférée de réalisation du procédé de l'invention, SiO₂ est présent dans le verre à une teneur allant de 40% à 55% en poids, par rapport au poids total du verre, CaO est présent dans le verre à une teneur allant de 15% à 25% en poids, par rapport au poids total du verre, Na₂O est présent dans le verre à une teneur allant de 15% à 25% en poids, par rapport au poids total du verre et P₂O₅ est présent dans le verre à une teneur allant de 1 % à 9% en poids, par rapport au poids total du verre.

K₂O peut par exemple être présent dans le verre à une teneur allant de 0,1 à 8% en poids, par rapport au poids total du verre. MgO peut par exemple être présent dans le verre à une teneur allant de 0,1 à 5% en poids, par rapport au poids total du verre. Al₂O₃ peut par exemple être présent dans le verre à une teneur allant de 0,1 à 1,5% en poids, par rapport au poids total du verre. CaF₂ peut par exemple être présent dans le verre à une teneur allant de 0,1 à 12,5% en poids, par rapport au poids total du verre. B₂O₃ peut par exemple être présent dans le verre à une teneur allant de 0,1 à 10% en poids, par rapport au poids total du verre.

De préférence, ce verre est constitué de 45% en poids de SiO₂, par rapport au poids total du verre, de 24,5% en poids de CaO, par rapport au poids total du verre, de 24,5% en poids de Na₂O, par rapport au poids total du verre et de 6% en poids de P₂O₅, par rapport au poids total du verre. Un tel verre est disponible commercialement sous la dénomination « 4555® » auprès de la société ORTHOVITA.

Alternativement, le verre de l'étape a°) peut être fabriqué selon toute méthode connue de fabrication d'un verre, comme par exemple la méthode sol-gel ou encore par fusion simple. La méthode sol-gel comprend classiquement une étape de mélange en solution des oxydes métalliques, puis une étape d'hydrolyse, suivie d'une gélification et d'un traitement à une température allant généralement de 600°C à 900°C pour produire un verre. La méthode par fusion comprend classiquement une étape de mélange des poudres d'oxydes, fusion et homogénéisation du mélange à une température allant généralement de 1250°C à 1600°C, puis refroidissement pour obtenir un verre amorphe.

Dans une deuxième étape, étape b°), le verre de l'étape a°) est broyé et tamisé pour obtenir une poudre. De préférence, cette poudre présente une masse volumique allant de 2, 5 g/cm³ à 3 g/cm³, de préférence de 2,6 g/cm³ à 2,8 g/cm³. De préférence, la taille moyenne des particules de la poudre va de 0,1 à 100 microns. De préférence, la température de transition vitreuse de la poudre va de 500°C à 700°C. De préférence, la température de cristallisation de la poudre va de 600°C à 900°C. De préférence, la température de fusion de la poudre va de 900°C à 1200°C.

Dans une troisième étape, étape c°), la poudre obtenue ci-dessus est mise en suspension dans un solvant et dispersée à l'aide d'un dispersant pour obtenir une barbotine. De préférence, le solvant est l'éthanol. De préférence, le taux de matière sèche dans la suspension va de 50% à 60% en poids, par rapport au poids de la suspension. De préférence, le dispersant est l'acide polyacrylique de sodium.

Dans une quatrième étape, étape d°), un agent épaississant est rajouté à la barbotine. De préférence, cet agent épaississant est une émulsion acrylique. Cet agent épaississant permet d'augmenter la viscosité de la barbotine. L'agent épaississant permet également d'éviter la sédimentation ou la flottation des particules d'agent porogène, dans le cas où un agent porogène est rajouté, comme décrit ci-après.

Dans une cinquième étape, étape e°), le mélange obtenu en d°) est coulé dans un moule puis séché pour donner un « gâteau ». De préférence, le séchage se fait à température ambiante.

Dans une sixième étape, étape f°), le « gâteau » obtenu en e°) est chauffé à une température d'environ 600°C pour obtenir un « cru ». Ce traitement thermique permet d'éliminer les ajouts organiques, tels que le dispersant, l'agent épaississant et les éventuels agents porogènes ajoutés. Ce corps en cru présente des macropores.

Dans une dernière étape, étape g°), le cru obtenu en f°) est traité à une température allant de 600°C à 1100°C, de préférence de 900°C à 1100°C, afin d'obtenir une cristallisation partielle ou totale de la composition. Ce dernier traitement permet d'obtenir une structure cristalline ou partiellement cristalline, ou encore vitro-céramique, poreuse massive.

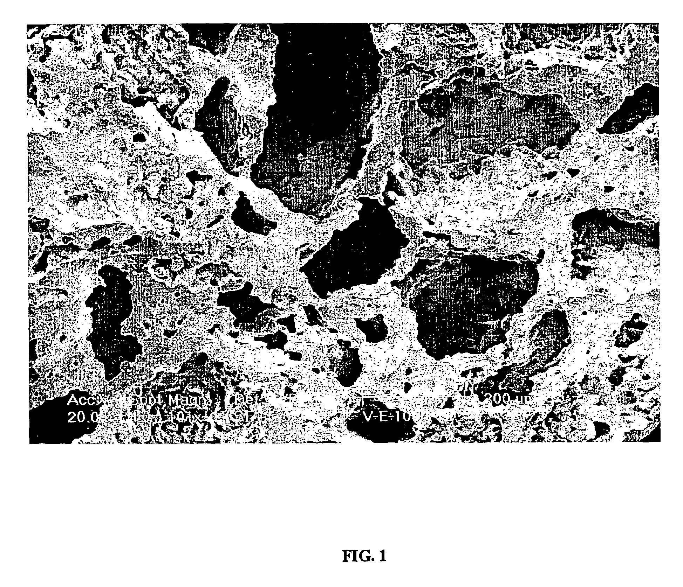

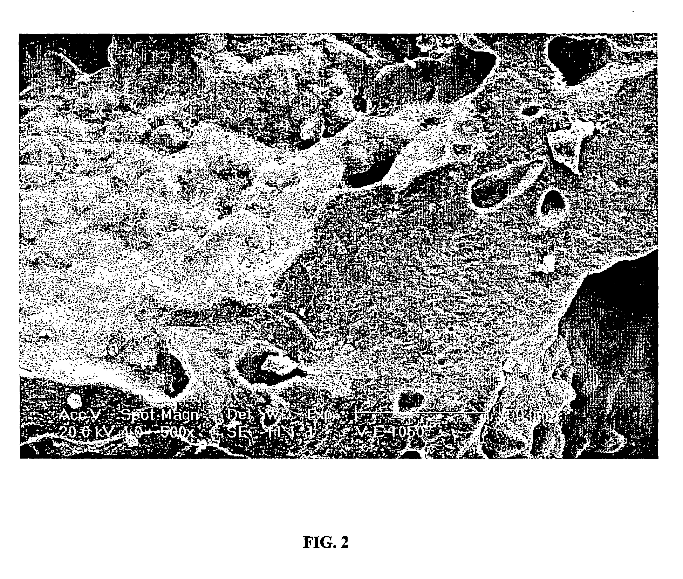

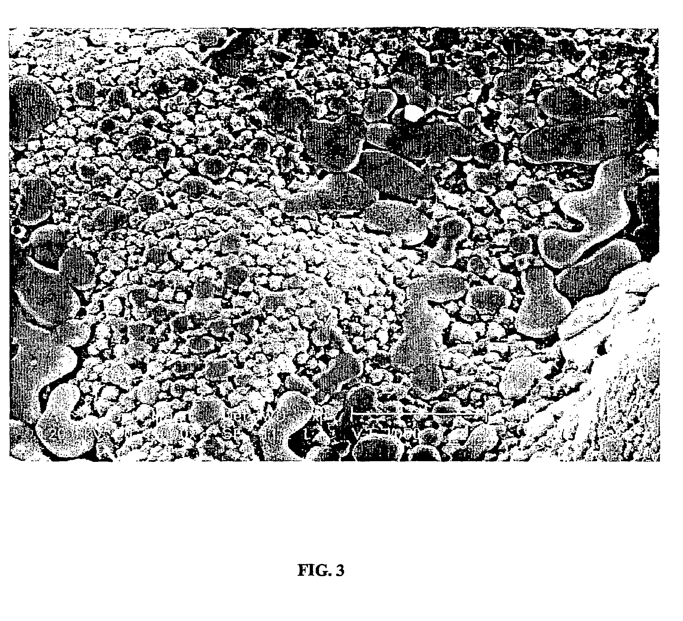

Dans une forme de réalisation du procédé de l'invention, le cru est traité à 900°C. La structure de la composition finale peut être observée sur les figures 1 à 3. On constate une multitude de pores de différentes tailles. La composition finale présente alors un taux de porosité d'environ 76%, mesuré selon la méthode géométrique.

Dans une autre forme de réalisation du procédé de l'invention, le cru est traité à 1000°C. La composition finale présente alors un taux de porosité d'environ 64%, mesuré selon la méthode géométrique.

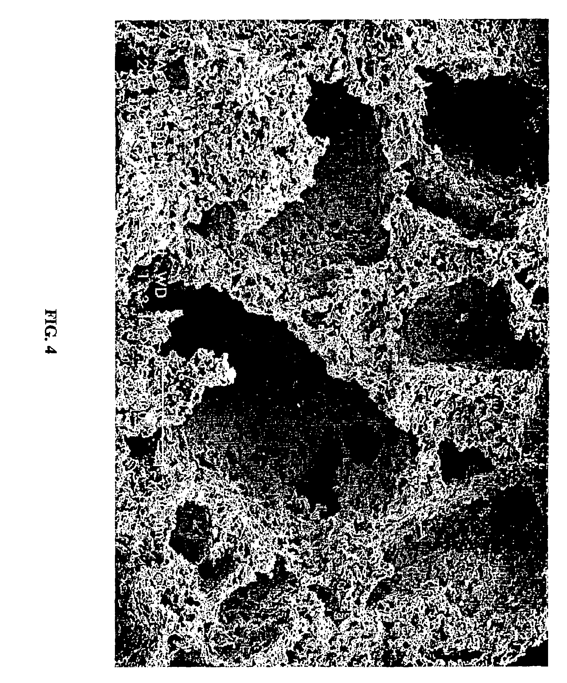

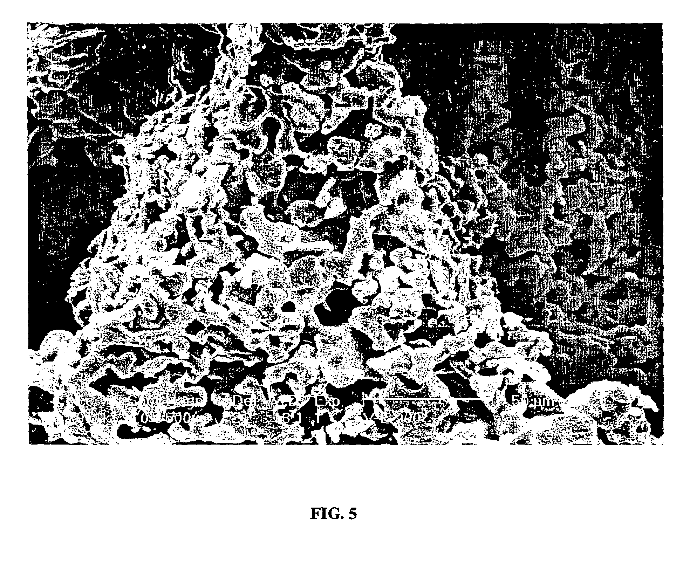

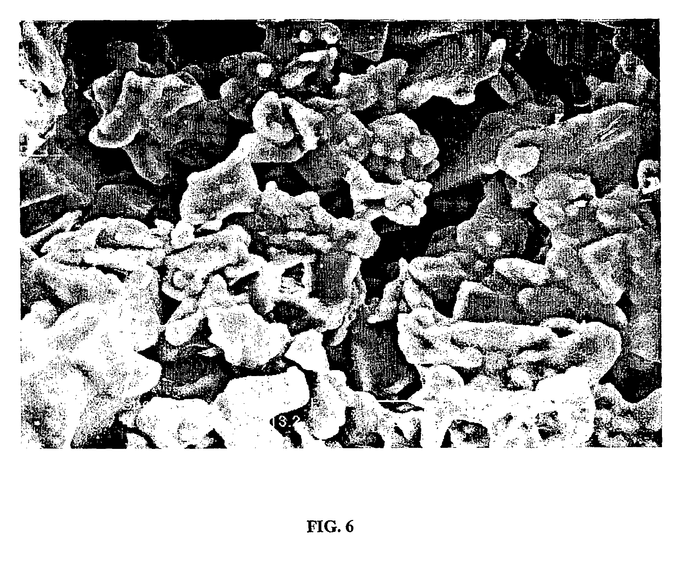

Dans une autre forme de réalisation du procédé de l'invention, le cru est traité à 1100°C. La structure de la composition finale peut être observée sur les figures 4 à 6. On constate que cette structure est plus dense que celle des figures 1 à 3. La composition finale présente alors un taux de porosité d'environ 60%, mesuré selon la méthode géométrique.

Dans une forme préférée de réalisation du procédé selon l'invention, un agent porogène est rajouté sous mélange à la barbotine, entre l'étape c°) et l'étape d°). Par agent porogène, on entend un composé que l'on introduit dans le mélange, sous forme de particules, ces particules se dégradant ensuite à haute température pour laisser des vides au sein du mélange, autrement dit des pores. De préférence, l'agent porogène est ajouté à un taux allant de 1% à 80% en volume, de préférence encore de 50% à 80%, en volume, par rapport au volume total de la barbotine. De préférence, l'agent porogène est choisi parmi un mélange de saccharose et d'amidon, le polyéthylène glycol, et leurs mélanges. De préférence, le mélange de saccharose et d'amidon est constitué de 80% en poids de saccharose et de 20% en poids d'amidon, par rapport au poids du mélange. De préférence, la taille des particules de l'agent porogène est inférieure ou égale à 1250 microns.

Dans une forme préférée de réalisation de l'invention, l'agent porogène est constitué d'un mélange de deux populations de tailles de particules. De préférence, ce mélange est constitué de 70% en volume, par rapport au volume total du mélange, de porogènes de tailles de particules inférieures à 630 microns et de 30% en volume, par rapport au volume total du mélange, de porogènes de tailles de particules allant de 630 à 1250 microns.

Ainsi, de préférence, la composition selon l'invention présente des macropores créées par la dégradation des particules d'agent porogène.

Quel que soit le taux de porosité obtenu, les propriétés mécaniques de la composition selon l'invention sont particulièrement excellentes, en particulier la résistance en compression. En effet, grâce au procédé de préparation particulier des compositions selon l'invention, les parois des interconnections sont bien soudées.

En particulier, la résistance à la compression des compositions selon l'invention est bien supérieure à celle de compositions de l'art antérieur à base d'hydroxyapatite et de phosphate tricalcique.

Après le traitement thermique, le cru peut être découpé afin de donner à l'implant osseux sa forme définitive. Le bloc, ou implant, est ensuite nettoyé à l'alcool dans une cuve à ultrasons, puis conditionné en salle blanche et stérilisé par irradiation γ.

Un autre objet de la présente invention est une composition telle que ci-dessus traitée par une solution de liquide physiologique de composition ionique proche de celle du plasma humain et de pH allant de 7 à 8, de préférence de 7,25 à 7,42.

De préférence, la composition ionique de ce liquide physiologique est la suivante :
- Na⁺ : 142,0 mmol/l,
- K⁺ : 5,0 mmol/l,
- Mg²⁺ : 1,5 mmol/l,
- Ca²⁺ : 2,5 mmol/l,
- Cl⁻ : 147,8 mmol/l,
- HCO₃⁻ : 4,2 mmol/l,
- HPO₄²⁻ : 1,0 mmol/l,
- SO₄²⁻ : 0,5 mmol/l.

De préférence, la composition selon l'invention est immergée dans cette solution à environ 37°C pendant une durée pouvant aller de 1 à 10 jours. On observe le développement d'une couche d'hydroxyapatite à la surface de la composition pendant cette immersion. On observe également une évolution de la microstructure de la composition par la création de nanopores, le diamètre moyen de ces nanopores étant inférieur ou égal à 1 micron, de préférence allant de 100 à 300 nanomètres.

Ainsi, dans une forme préférée de réalisation de l'invention, la composition est traitée par une solution de liquide physiologique de composition ionique proche de celle du plasma humain et de pH allant de 7 à 8, de préférence de 7,25 à 7,42, et présente une couche d'hydroxyapatite cristallisée à sa surface. De préférence encore, cette composition traitée présente des nanopores, le diamètre moyen de ces nanopores étant inférieur ou égal à 1 micron, de préférence allant de 100 à 300 nanomètres.

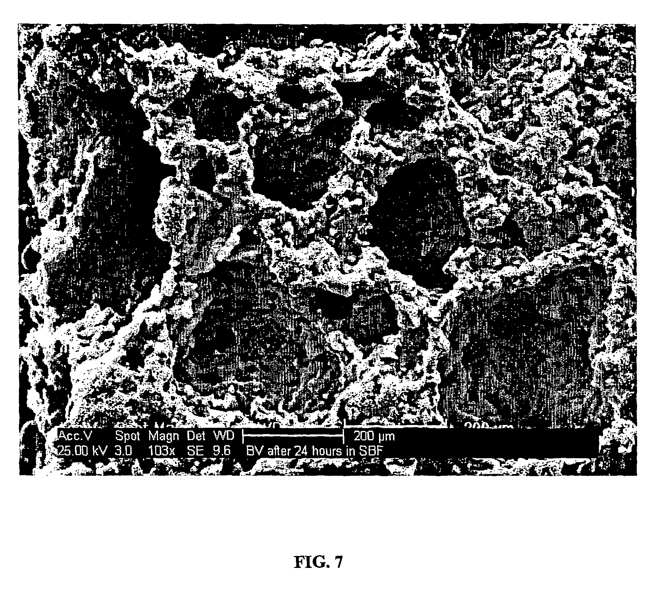

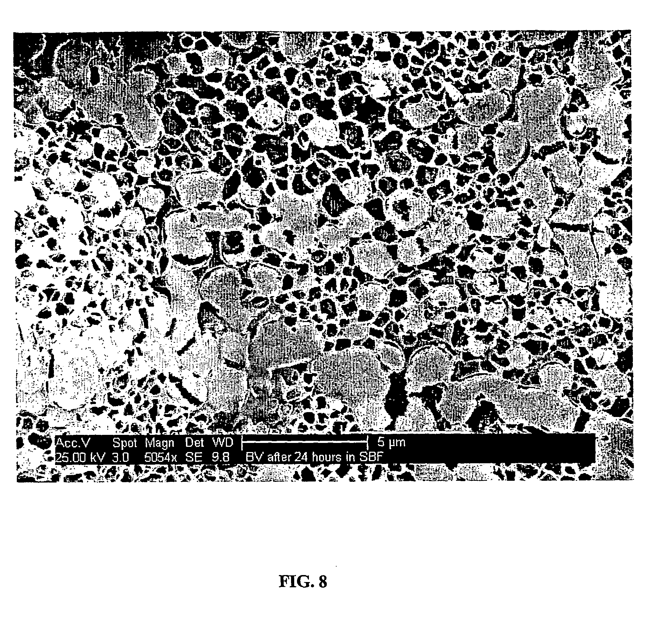

Sur les figures 7 et 8, on peut observer la structure d'une composition, obtenue selon le procédé de l'invention dans lequel le traitement de l'étape g°) a été effectué à 1100°C, puis immergée dans une solution de liquide physiologique de composition ionique telle que décrite ci-dessus pendant 24h. On constate la formation d'une couche d'hydroxyapatite à la surface de la composition (figure 7) ainsi que la formation de nanopores de diamètre moyen inférieur à 1 micron (figure 8).

Un exemple illustrant la présente invention va maintenant être donné.

### EXEMPLE 1 (selon l'invention) :

Une composition comprenant 45% en poids de SiO₂, par rapport au poids total de la composition, 24,5% en poids de CaO, par rapport au poids total de la composition, 24,5% en poids de Na₂O, par rapport au poids total de la composition et 6% en poids de P₂O₅, par rapport au poids total de la composition est préparée de façon classique par fusion.
100 g de cette composition est broyée et tamisée pour obtenir une poudre. Les caractéristiques de la poudre sont :
   - Masse volumique (g/cm3) : 2,75
   - Diamètre des particules (microns) : 5
   - Température de transition vitreuse Tg (°C) : 538
   - Température de cristallisation Tc (°C) : 777
   - Température de fusion Tf (°C) : 1060
200 g d'une barbotine de caractéristiques suivantes est préparée à partir de cette poudre :
   - taux de poudre (% en masse) : 50%
   - dispersant : acide polyacrylique de sodium
   - taux de dispersant (en masse) : 0,5%
   - agent épaississant : émulsion acrylique
   - taux d'agent épaississant (en masse): 2 à 3%
   - agent porogène : polyéthylène glycol
   - taux de porogène (en volume): 80%
   - taille des particules de porogène : mélange de 70% de tailles inférieures à 630 microns et 30% de tailles allant de 630 microns à 1250 microns.
   - Solvant : éthanol

La barbotine est préparée avec l'éthanol et dispersée puis mélangée dans un mélangeur mécanique (broyeur à boulets) dans des poudriers en polyéthylène. Le polyéthylène glycol est ensuite rajouté, puis l'agent épaississant.

Le mélange est ensuite coulé dans un moule pour donner, après séchage à l'ambiante, un gâteau qui est ensuite démoulé. Le gâteau est ensuite traité à 600°C pour éliminer les ajouts organiques, à savoir le dispersant, l'agent épaississant et le porogène. Le cru est ensuite traité à une température de 1100°C. On obtient une composition cristalline poreuse massive de caractéristiques suivantes :

Le taux de porosité de la composition finale est : 75%, mesuré selon la méthode géométrique.

La résistance en compression de la composition finale est : 7 MPa, mesurée selon la méthode décrite dans la présente demande.

Le diamètre moyen des macropores est : 100-1250 microns.

Le diamètre moyen des micropores est : inférieur à 5 microns.

Un substitut osseux, ou implant, est découpé dans cette composition cristalline massive sans que cette dernière ne s'effrite.

A titre de comparaison, un substitut osseux constitué de 70% en poids, par rapport au poids total du substitut, d'hydroxyapatite, et de 30% en poids, par rapport au poids total du substitut, de phosphate tricalcique β, présente une résistance en compression de l'ordre de 1,5 MPa, mesurée selon la méthode décrite dans la présente demande, pour un taux de porosité totale, mesuré selon la méthode géométrique, de 73% et pour un diamètre moyen des pores strictement inférieur à 600 microns.

## Revendications

1. Procédé de préparation d'une composition cristalline ou partiellement cristalline poreuse massive comprenant au moins SiO₂, CaO, Na₂O, et P₂O₅, comprenant des micropores et des macropores, dont le taux de porosité va de 50% à 80% mesuré par la méthode géométrique, le diamètre moyen des macropores allant de 100 à 1250 micromètres, de préférence de 150 micromètres à 300 micromètres, le diamètre moyen des micropores étant inférieur ou égal à 5 micromètres, **caractérisé en ce qu'**il comprend les étapes suivantes:
- a°) on dispose d'un verre à base de SiO₂, CaO, Na₂O, et P₂O₅,
- b°) le verre de l'étape a°) est broyé et tamisé pour obtenir une poudre,
- c°) la poudre obtenue en b°) est mise en suspension dans un solvant et dispersée à l'aide d'un dispersant pour obtenir une barbotine,
- d°) un agent épaississant est rajouté à la barbotine obtenue en c°),
- e°) le mélange obtenu en d°) est coulé dans un moule puis séché pour donner un « gâteau »,
- f°) le « gâteau » obtenu en e°) est chauffé à une température d'environ 600°C pour obtenir un « cru »,
- g°) le cru obtenu en f°) est traité à une température allant de 600°C à 1100°C, de préférence de 900°C à 1100°C, afin d'obtenir une cristallisation partielle ou totale de la composition,
un agent porogène étant rajouté sous mélange à la barbotine, entre l'étape c°) et l'étape d°).

2. Procédé selon la revendication précédente, **caractérisé en ce que** l'agent épaississant est une émulsion acrylique.

3. Procédé selon la revendication 1 ou 2, **caractérisé en ce que** le cru est traité à 900°C.

4. Procédé selon la revendication 1 ou 2, **caractérisé en ce que** le cru est traité à 1000°C.

5. Procédé selon la revendication 1 ou 2, **caractérisé en ce que** le cru est traité à 1100°C.

6. Procédé selon l'une quelconque des revendications 1 à 5, **caractérisé en ce que** l'agent porogène est ajouté à un taux allant de 1 % à 80% en volume, de préférence encore de 50% à 80%, en volume, par rapport au volume total de la barbotine.

7. Procédé selon la revendication 5 ou 6, **caractérisé en ce que** l'agent porogène est choisi parmi un mélange de saccharose et d'amidon, le polyéthylène glycol, et leurs mélanges.

8. Procédé selon l'une quelconque des revendications 5 à 7, **caractérisé en ce que** l'agent porogène est constitué d'un mélange de deux populations de tailles de particules.

9. Procédé selon la revendication précédente, **caractérisé en ce que** ce mélange est constitué de 70% en volume, par rapport au volume total du mélange, de porogènes de tailles de particules inférieures à 630 micromètres et de 30% en volume, par rapport au volume total du mélange, de porogènes de tailles de particules allant de 630 à 1250 micromètres.

10. Composition susceptible d'être obtenue selon le procédé selon l'une quelconque des revendications 1 à 9.

11. Composition selon la revendication 10, **caractérisée en ce qu'**elle est traitée par une solution de liquide physiologique de composition ionique proche de celle du plasma humain.

12. Composition selon la revendication précédente, **caractérisée en ce que** la composition ionique de ce liquide physiologique est la suivante :
- Na⁺ : 142,0 mmol/l,
- K⁺ : 5,0 mmol/l,
- Mg²⁺ : 1,5 mmol/l,
- Ca²⁺ : 2,5 mmol/l,
- Cl⁻ : 147,8 mmol/l,
- HCO₃⁻ : 4,2 mmol/l,
- HPO₄²⁻ : 1,0 mmol/l,
- SO₄²⁻ : 0,5 mmol/l.

13. Composition selon la revendication 11 ou 12, **caractérisée en ce qu'**elle présente une couche d'hydroxyapatite à sa surface.

14. Composition selon l'une quelconque des revendications 11 à 13, **caractérisée en qu'**elle présente des nanopores, le diamètre moyen de ces nanopores étant inférieur ou égal à 1 micromètre, de préférence allant de 100 à 300 nanomètres.

15. Implant **caractérisé en ce qu'**il est constitué d'une composition selon l'une quelconque des revendications 10 à 14.

## Claims

1. A method for preparing a bulk porous, crystalline or partially crystalline composition comprising at least SiO₂, CaO, Na₂O and P₂O₅, comprising micropores and macropores, of which the degree of porosity ranges from 50% to 80% measured by the geometric method, the average diameter of the macropores ranging from 100 to 1250 micrometers, preferably from 150 micrometers to 300 micrometers, the average diameter of the micropores being less than or equal to 5 micrometers, **characterized in that** it comprises the following steps:
- a) a glass based on SiO₂, CaO, Na₂O and P₂O₅ is used;
- b) the glass from step a) is ground and sieved to obtain a powder;
- c) the powder obtained in b) is suspended in a solvent and dispersed using a dispersant in order to obtain a slurry;
- d) a thickening agent is added to the slurry obtained in c);
- e) the mixture obtained in d) is poured into a mold then dried to give a "cake";
- f) the "cake" obtained in e) is heated at a temperature of about 600°C in order to obtain a "green body"; and
- g) the green body obtained in f) is treated at a temperature ranging from 600°C to 1100°C, preferably from 900°C to 1100°C, in order to obtain partial or total crystallization of the composition,
a pore-forming agent being added with mixing to the slurry, between step c) and step d).

2. The method as claimed in the preceding claim, **characterized in that** the thickening agent is an acrylic emulsion.

3. The method as claimed in claim 1 or 2, **characterized in that** the green body is treated at 900°C.

4. The method as claimed in claim 1 or 2, **characterized in that** the green body is treated at 1000°C.

5. The method as claimed in claim 1 or 2, **characterized in that** the green body is treated at 1100°C.

6. The method as claimed in anyone of claims 1-5, **characterized in that** the pore-forming agent is added in an amount ranging from 1% to 80% by volume, more preferably from 50% to 80% by volume, relative to the total volume of the slurry.

7. The method as claimed in claim 5 or 6, **characterized in that** the pore-forming agent is chosen from a mixture of saccharose and starch, polyethylene glycol and mixtures thereof.

8. The method as claimed in any one of claims 5 to 7, **characterized in that** the pore-forming agent consists of a mixture of two particle size populations.

9. The method as claimed in the preceding claim, **characterized in that** this mixture consists of 70% by volume, relative to the total volume of the mixture, of pore-forming agents with particle sizes of less than 630 micrometers and of 30% by volume, relative to the total volume of the mixture, of pore-forming agents with particle sizes ranging from 630 to 1250 micrometers.

10. A composition capable of being obtained according to the method as claimed in any one of claims 1 to 9.

11. The composition as claimed in claim 10, **characterized in that** it is treated with a solution of a physiological fluid with an ionic composition close to that of human plasma.

12. The composition as claimed in the preceding claim, **characterized in that** the ionic composition of this physiological fluid is the following:
- Na⁺ : 142.0 mmol/l;
- K⁺ : 5.0 mmol/l;
- Mg²⁺ : 1.5 mmol/l;
- Ca²⁺ : 2.5 mmol/l;
- Cl⁻ : 147.8 mmol/l;
- HCO₃⁻ : 4.2 mmol/l;
- HPO₄²⁻ : 1.0 mmol/l and
- SO₄²⁻ : 0.5 mmol/l.

13. The composition as claimed in claim 11 or 12, **characterized in that** it has a hydroxyapatite layer on its surface.

14. The composition as claimed in any one of claims 11 to 13, **characterized in that** it has nanopores, the average diameter of these nanopores being less than or equal to 1 micrometer, preferably ranging from 100 to 300 nanometers.

15. An implant **characterized in that** it is made from a composition as claimed in any one of claims 10 to 14.

## Patentansprüche

1. Verfahren zur Herstellung einer massiven porösen kristallinen oder teilweise kristallinen Zusammensetzung, die zumindest SiO₂, CaO, Na₂O und P₂O₅ mit Mikroporen und Makroporen aufweist, deren durch die geometrische Methode gemessener Porositätsgrad 50 % bis 80 % beträgt, wobei der durchschnittliche Durchmesser der Makroporen 100 bis 1250 Mikrometer, vorzugsweise 150 Mikrometer bis 300 Mikrometer, beträgt, wobei der durchschnittliche Durchmesser der Mikroporen kleiner oder gleich 5 Mikrometer ist, **dadurch gekennzeichnet, dass** es die folgenden Schritte umfasst:
- a) man verfügt über ein Glas auf der Basis von SiO₂, CaO, Na₂O und P₂O₅,
- b) das Glas von Schritt a) wird zerkleinert und gesiebt, um ein Pulver zu erhalten,
- c) das in Schritt b) hergestellte Pulver wird in einem Lösungsmittel suspendiert und mit Hilfe eines Dispersionsmittels dispergiert, um einen Schlicker zu erhalten,
- d) dem in c) hergestellten Schlicker wird ein Verdickungsmittel hinzugefügt,
- e) das in d) hergestellte Gemisch wird in eine Form gegossen und danach getrocknet, um einen "Kuchen" zu bilden,
- f) der in e) hergestellte "Kuchen" wird auf eine Temperatur von zirka 600 °C erhitzt, um einen "Rohling" zu erhalten,
- g) der in f) hergestellte Rohling wird bei einer Temperatur von 600 °C bis 1100 °C, vorzugsweise von 900 °C bis 1100 °C, behandelt, um eine teilweise oder vollständige Kristallisation der Zusammensetzung zu erhalten,
wobei dem Schlicker zwischen Schritt c) und Schritt d) ein Blähmittel hinzugemischt wird.

2. Verfahren nach vorangehendem Anspruch, **dadurch gekennzeichnet, dass** das Verdickungsmittel eine Acrylemulsion ist.

3. Verfahren nach Anspruch 1 oder 2, **dadurch gekennzeichnet, dass** der Rohling bei 900 °C bearbeitet wird.

4. Verfahren nach Anspruch 1 oder 2, **dadurch gekennzeichnet, dass** der Rohling bei 1000 °C bearbeitet wird.

5. Verfahren nach Anspruch 1 oder 2, **dadurch gekennzeichnet, dass** der Rohling bei 100°C bearbeitet wird.

6. Verfahren nach einem der Ansprüche 1 bis 5, **dadurch gekennzeichnet, dass** das Blähmittel in einem Anteil von 1 bis 80 Vol.-%, vorzugsweise auch von 50 bis 80 Vol.-%, im Verhältnis zum Gesamtvolumen des Schlickers hinzugefügt wird.

7. Verfahren nach Anspruch 5 oder 6, **dadurch gekennzeichnet, dass** das Blähmittel aus einem Gemisch von Saccharose und Stärke, dem Polyethylenglycol und ihren Gemischen ausgewählt ist.

8. Verfahren nach einem der Ansprüche 5 bis 7, **dadurch gekennzeichnet, dass** das Blähmittel aus einem Gemisch von zwei Partikelgrößenpopulationen besteht.

9. Verfahren nach vorangehendem Anspruch, **dadurch gekennzeichnet, dass** dieses Gemisch aus 70 Vol.-%, im Verhältnis zum Gesamtvolumen des Gemischs, Blähmittel mit einer Partikelgröße unter 630 Mikrometer und aus 30 Vol.-%, im Verhältnis zum Gesamtvolumen des Gemischs, Blähmittel mit einer Partikelgröße von 630 bis 1250 Mikrometer besteht.

10. Zusammensetzung, die nach dem Verfahren nach einem der Ansprüche 1 bis 9 herstellbar ist.

11. Zusammensetzung nach Anspruch 10, **dadurch gekennzeichnet, dass** sie mit einer physiologischen Flüssigkeitslösung mit einer dem menschlichen Plasma ähnlichen Ionenzusammensetzung behandelt wird.

12. Zusammensetzung nach vorangehendem Anspruch, **dadurch gekennzeichnet, dass** die Ionenzusammensetzung dieser physiologischen Flüssigkeit folgende ist.
- Na⁺: 142,0 mmol/l,
- K⁺: 5,0 mmol/l,
- Mg²⁺: 1,5mmol/l,
- Ca²⁺: 2,5 mmol/l,
- Cl⁻: 147,8 mmol/l,
- HCO₃⁻: 4,2 mmol/l,
- HPO₄²⁻: 1,0 mmol/l,
- SO₄²⁻: 0,5 mmol/l.

13. Zusammensetzung nach Anspruch 11 oder 12, **dadurch gekennzeichnet, dass** sie auf ihrer Oberfläche eine Hydroxyapatitschicht aufweist.

14. Zusammensetzung nach einem der Ansprüche 11 bis 13, **dadurch gekennzeichnet, dass** sie Nanoporen aufweist, wobei der durchschnittliche Durchmesser dieser Nanoporen kleiner oder gleich 1 Mikrometer ist, vorzugsweise 100 bis 300 Nanometer.

15. Implantat, **dadurch gekennzeichnet, dass** es aus einer Zusammensetzung nach einem der Ansprüche 10 bis 14 besteht.
